# EUROPÄISCHE PATENTSCHRIFT

(11) **EP 1 162 197 B1**
(45) Veröffentlichungstag und Bekanntmachung des Hinweises auf die Patenterteilung: **26.05.2004**
(21) Anmeldenummer: 01112068.0
(22) Anmeldetag: 28.05.2001
(51) Int. Cl.: C07D 213/803, C07D 213/80

(54) **Verfahren zur Herstellung von Pyridinen durch Oxidation von Dihydropyridinen mittels Methylnitrit**
Process for the preparation of pyridines by oxidation of dihydropyridines using methyl nitrite
Procédé pour la préparation des pyridines par oxidation des dihydropyridines en utilisant methyl nitrite

(30) Priorität: 08.06.2000 DE 10028414; 13.03.2001 DE 10111874
(43) Veröffentlichungstag der Anmeldung: 12.12.2001
(73) Patentinhaber: Bayer Chemicals AG, 51368 Leverkusen (DE)
(72) Erfinder: Lui, Norbert, Dr., 51061 Köln (DE); Panskus, Hans, 51373 Leverkusen (DE); Schnatterer, Albert, Dr., 51373 Leverkusen (DE)

(56) Entgegenhaltungen:
- WO-A-99/19302
- HOUBEN-WEYL: "Methoden der organischen Chemie, Bd.IV/1a" 1981 , GEROG THIEME VERLAG , STUTTGART XP002174903 * Seite 839 - Seite 846 * * Tabelle 18, Seite 844 oberster Eintrag *
- A. HANTZSCH: "Ueber die Synthese pyridinartiger Verbindungen aus Acetessigäther und Aldehydammoniak" JUSTUS LIEBIGS ANNALEN DER CHEMIE, Bd. 215, 1882, Seiten 1-81, XP001013491

## Beschreibung

Die vorliegende Erfindung betrifft ein verbessertes Verfahren zur Herstellung von substituierten Pyridinen durch Oxidation (Aromatisierung) der entsprechenden 1,4-Dihydropyridine.

Substituierte Pyridine sind wichtige Produkte zur Herstellung von Pharmazeutika, Pflanzenschutzmitteln und Farbstoffen.

Für die Oxidation (Aromatisierung) von 1,4-Dihydropyridinen sind viele Oxidationsmittel bekannt, beispielsweise Eisen- und Kupfernitrate auf Trägem, gegebenenfalls unter Ultraschalleinwirkung, Cerammoniumnitrat, Pyridiniumchlorochromat, Salpetersäure und Stickstoffmonoxid (siehe J. Org. Chem. 62, 3582 (1997)). Diese Oxidationsverfahren sind teils teuer, teils erfordern sie hohen apparativen und sicherheitstechnischen Aufwand, teils erfordern sie ökologisch bedenkliche Oxidationsmittel. Das in dieser Literaturstelle als Oxidationsmittel empfohlene Stickstoffmonoxid muss unter Argon gehandhabt werden, weil es mit Luftsauerstoff reagiert und ist nicht kostengünstig zugänglich. Gemäß J. Med. Chem. 29, 1596 (1986) wird mit Salpetersäure oxidiert, wobei große Mengen Salpetersäure nötig sind (auf 0,66 g Edukt 12 ml wässrige Salpetersäure), und das Produkt durch 3-fache Extraktion mit Ether isoliert werden muss.

Weiterhin ist aus WO-A 99/19302 und Hantsch, Justus Liebigs Annalen der Chemie, Band 215, 1882, Seiten 1 bis 81 (auch zitiert in Houben-Weyl, Methoden der organischen Chemie, Band IV/1a, 1981, Georg Thieme Verlag, Stuttgart) bekannt, Salpetersäure oder salpetrige Säure als Oxidationsmittel einzusetzen. Diese Verfahren besitzen jedoch den Nachteil, dass die eingesetzten Oxidantien stark korrodierend wirken und somit einen hohen apparativen Aufwand erforderlich machen.

Für die Herstellung von 4-(4-Fluorphenyl)-2,6-diisopropyl-3,5-carbonsäuremethylester-pyridin ist die Oxidation des entsprechenden 1,4-Dihydropyridins mit Schwefel bekannt (siehe US-A 4 950 675). Neben der problematischen Handhabung von Schwefel ist bei diesem Verfahren außerordentlich nachteilig, dass es nur Ausbeuten von deutlich unter 40 % ermöglicht und hochgiftiger Schwefelwasserstoff als Nebenprodukt entsteht.

Es steht also noch kein einfach durchführbares, kostengünstiges und hohe Ausbeuten lieferndes Verfahren zur Herstellung von substituierten Pyridinen durch Oxidation der entsprechenden 1,4-Dihydropyridine zur Verfügung.

Es wurde nun ein Verfahren zur Herstellung von substituierten Pyridinen der Formel (I) gefunden 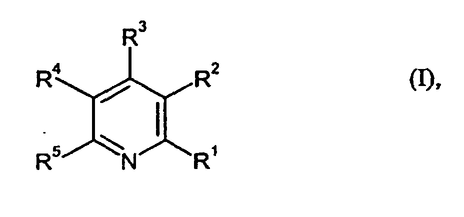 in der
- R¹ und R⁵: gleich oder verschieden sind und jeweils für C₁-C₁₀-Alkyl oder C₆-C₁₀-Aryl stehen,
- R² und R⁴: gleich oder verschieden sind und jeweils für Wasserstoff, C₁-C₁₀-Alkyl, CN oder COOR⁶ mit R⁶ = C₁-C₁₀-Alkyl stehen und
- R³: für Wasserstoff, C₁-C₁₀-Alkyl oder gegebenenfalls durch Halogen, Nitro, COOR⁶ (R⁶ wie oben definiert), CN oder C₁-C₁₀-Alkyl substituiertes C₆-C₁₀-Aryl steht,
das dadurch gekennzeichnet ist, dass man ein substituiertes 1,4-Dihydropyridin der Formel (II) 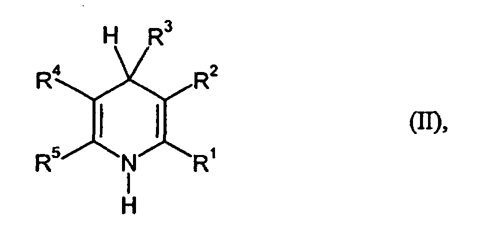 in der
- R¹ bis R⁵: die bei Formel (I) angegebene Bedeutung haben,
mit Methylnitrit in Gegenwart von Säure umsetzt, die weniger als 20 Gew.-% oxidierende Bestandteile enthält.

In den Formeln (I) und (II)
- sind R¹ und R⁵ vorzugsweise gleich und stehen vorzugsweise jeweils für geradkettiges oder verzweigtes C₁-C₆-Alkyl,
- sind R² und R⁴ vorzugsweise gleich und stehen vorzugsweise jeweils für COOR⁶ mit R⁶ = geradkettigem oder verzweigtem C₁-C₆-Alkyl und
- steht R³ vorzugsweise für mit Fluor- und/oder Chlor substituiertes Phenyl.

In den Formeln (I) und (II)
- stehen R¹ und R⁵ besonders bevorzugt für Isopropyl,
- stehen R² und R³ besonders bevorzugt für COOR⁶ mit R⁶ = Methyl oder Ethyl und
- steht R³ besonders bevorzugt für 4-Fluorphenyl.

Das benötigte Methylnitrit kann auf einfache, an sich bekannte Weise, durch Umsetzung von Alkalinitriten mit Methanol in Gegenwart einer starken Säure hergestellt werden. Es fällt bei dieser Herstellungsmethode gasförmig an und kann in dieser Form in das erfindungsgemäße Verfahren eingesetzt werden. Es ist vorteilhaft, das Methylnitrit wenigstens in der stöchiometrisch erforderlichen Menge einzusetzen. Auch größere Überschüsse an Methylnitrit stören nicht. Vorzugsweise setzt man 1 bis 20 Mol Methylnitrit pro Mol 1,4-Dihydropyridin der Formel (II) ein.

Als Säuren kommen die verschiedensten anorganischen und organischen Säuren in Frage. Bevorzugt sind Mineral- und Carbonsäuren. Besonders bevorzugt sind gasförmiger Chlorwasserstoff, wässrige Salzsäure, wässrige Schwefelsäure und C₁-C₄-Carbonsäure in Substanz oder wässriger Lösung. Die Menge der Säure kann in weiten Grenzen variiert werden. Beispielsweise kann man sie in katalytischen, stöchiometrischen oder überstöchiometrischen Mengen einsetzen. Vorzugsweise setzt man sie pro Mol 1,4-Dihydropyridin der Formel (II) in Mengen von 0,01 bis 2 Molen ein. Es können auch Gemische verschiedener Säuren eingesetzt werden. Carbonsäuren können auch als Lösungsmittel fungieren und deshalb auch in höheren Molverhältnissen umgesetzt werden. Beispielsweise bis zu 50 Mol, vorzugsweise bis zu 30 Mol, jeweils pro Mol 1,4-Dihydropyridin der Formel (II).

Die einzusetzende Säure enthält weniger als 20 Gew.-% oxidierende Bestandteile. Oxidierende Bestandteile können z.B. Salpetersäure oder Salze mit oxidierender Wirkung sein. Vorzugsweise liegt der Gehalt der Säure an oxidierenden Bestandteilen unter 5 Gew.-%. Besonders bevorzugt ist die Säure frei von oxidierenden Bestandteilen.

Man kann das 1,4-Dihydropyridin der Formel (II) auch in Form von 1,4-Dihydropyridinium-Salzen von nicht-oxidierenden Säuren einsetzen, z.B. in Form von 1,4-Dihydroxypyridin-hydrochloriden. In solchen Fällen kann auf den separaten Zusatz einer Säure verzichtet werden.

Wenn man die Säure in Form einer wässrigen Lösung in einer Menge einsetzt, dass das Reaktionsgemisch eine gut rührbare Suspension oder Lösung bildet, ist kein Zusatz an sonstigen Lösungsmitteln erforderlich. Sonst ist es angezeigt Lösungsmittel zuzusetzen. Als Lösungsmittel kommen solche in Frage, die keine unerwünschten Nebenreaktionen eingehen. Beispiele sind Wasser, aromatische Kohlenwasserstoffe wie Toluol, Alkohole wie Methanol, Ether wie Dibutylether, halogenierte Kohlenwasserstoffe wie Dichlorethan und Chlorbenzol, Sulfoxide wie Dimethylsulfoxid und Sulfone wie Tetramethylensulfon. Carbonsäuren können auch als Lösungsmittel fungieren (siehe oben).

Lösungsmittel setzt man gegebenenfalls in solchen Mengen ein, dass das Reaktionsgemisch eine gut rührbare Suspension oder Lösung bildet.

Es ist bevorzugt folgende Kombinationen von Säuren und Lösungsmittel anzuwenden: wässrige Salzsäure/Wasser, gasförmiger Chlorwasserstoff/Alkohol und Essigsäure/Wasser.

Das erfindungsgemäße Verfahren kann man auf verschiedene Weise durchführen. Beispielsweise kann man das 1,4-Dihydropyridin der Formel (II) in einem Lösungsmittel suspendieren oder lösen, dann die Säure zusetzen und dann gasförmiges Methylnitrit einleiten. Man kann auch das 1,4-Dihydropyridin der Formel (II) mit soviel einer wässrigen Lösung der Säure versetzen, dass eine gut rührbare Suspension oder Lösung entsteht und dann Methylnitrit gasförmig einleiten. Man kann die Säure auch simultan, aber getrennt von dem Methylnitrit zudosieren.

Weiterhin kann man z.B. auch 1,4-Dihydropyridin der Formel (II) in Wasser suspendieren, dann gasförmigen Chlorwasserstoff und anschließend Methylnitrit einleiten. Es sind auch noch andere Durchführungsmöglichkeiten für das erfindungsgemäße Verfahren denkbar.

Als Reaktionstemperaturen für das erfindungsgemäße Verfahren kommen z.B. solche zwischen -30 und +100°C, insbesondere solche von -10 bis +65°C in Frage.

Das nach beendeter Umsetzung vorliegende Reaktionsgemisch kann auf einfache Weise aufgearbeitet werden. Wenn man im wesentlichen in wässrigem Milieu gearbeitet hat, kann man das Reaktionsgemisch zunächst mit einer beliebigen Base neutralisieren und dann das hergestellte substituierte Pyridin der Formel (I) abtrennen, z.B. durch Filtration.

Wenn man im wesentlichen in alkoholischem Medium oder in einem anderen, mit Wasser mischbaren Lösungsmittel gearbeitet hat, kann man dem Reaktionsgemisch nach der Neutralisation mit einer beliebigen Base Wasser zufügen und das hergestellte substituierte Pyridin der Formel (I) abtrennen, z.B. durch Filtration.

Wenn man in einem mit Wasser nicht mischbaren Milieu gearbeitet hat, kann man aus dem Reaktionsgemisch nach der Neutralisation mit einer beliebigen Base das Lösungsmittel teilweise oder ganz entfernen und so das hergestellte substituierte Pyridin der Formel (I) erhalten.

Es sind auch noch andere einfache Möglichkeiten für die Aufarbeitung der Reaktionsgemische denkbar.

Bei der Base zur Neutralisation des Reaktionsgemisches kann es sich um beliebige Basen handeln, die keine unerwünschten Nebenreaktionen eingehen. Geeignet sind beispielsweise wässrige Alkalilaugen, Alkoholate als solche und in alkoholischer Lösung und Amine. Vorzugsweise verwendet man wässrige Alkalilaugen, wenn man die Reaktion in wässrigem Milieu durchgeführt hat. Alkoholate sind bevorzugt, wenn die Reaktion in einem alkoholischen Medium durchgeführt worden ist.

Bei Verwendung der Carbonsäure kann die Neutralisation gegebenenfalls unterbleiben.

Das erfindungsgemäße Verfahren hat eine Reihe von Vorteilen. So sind die erzielbaren Ausbeuten, die im allgemeinen zwischen 90 und 99 % der Theorie liegen, hoch, die Durchführung ist einfach, das Methylnitrit kann auf einfache Weise nach Bedarf hergestellt werden, es werden keine ökologisch bedenklichen Schwermetallsalze zur Oxidation verwendet, so entstehen keine hochgiftigen Nebenprodukte und die Aufarbeitung des Reaktionsgemisches ist einfach.

### Beispiele

### Beispiel 1 (nicht erfindungsgemäß)

Das in den nachfolgenden Beispielen eingesetzte Methylnitrit wurde nach Bedarf durch Behandlung einer Lösung aus 1 Gew.-Teil 25 gew.-%iger wässriger Natriumnitritlösung und 0,14 Gew.-Teilen Methanol mit 0,37 Gew.-Teilen 48 gew.-%iger wässriger Schwefelsäure hergestellt und gasförmig zur erfindungsgemäßen Oxidation (Aromatisierung) von substituierten 1,4-Dihydropyridinen der Formel (II) verwendet.

### Beispiel 2

In einem 4 l Glasreaktor wurden 750 g 4-(4-Fluorphenyl)-2,6-diisopropyl-3,5-carbonsäuremethylester-1,4-dihydropyridin in 2250g Wasser und 207g 37 gew.-%ige wässrige Salzsäure vorgelegt. Anschließend wurden bei 60°C 15 Mol Methylnitrit eingeleitet. Nach Abkühlung auf 20°C wurde mit 198 g 45 gew.-%iger wässriger Natronlauge ein pH-Wert von 8 eingestellt. Die dann vorliegende Suspension wurde filtriert, das abfiltrierte Produkt zweimal mit je 800 ml Wasser gewaschen und abschließend im Vakuum bei 55°C getrocknet. Es wurden 736 g 4-(4-Fluorphenyl)-2,6-diisopropyl-3,5-carbonsäuremethylester-pyridin isoliert, was einer Ausbeute von 98 % der Theorie entspricht.

### Beispiel 3

In einem 2 l Glasreaktor wurden 375 g 4-(4-Fluorphenyl)-2,6-diisopropyl-3,5-carbonsäuremethylester-1,4-dihydropyridin in 711,6 g Methanol bei -5°C gelöst. Anschließend wurden 4 g gasförmiger Chlorwasserstoff und 2 Mol Methylnitrit bei -3 bis -10°C eingeleitet. Nach dem Aufwärmen auf 20°C wurde durch Zufügen von 19,5 g 30 gew.-%iger methanolischer Natriummethylatlösung neutralisiert. Die Lösung wurde zum Sieden erhitzt und anschließend mit 170 g Wasser versetzt. Nach dem Abkühlen auf +5°C wurde die vorliegende Suspension filtriert, das abfiltrierte Produkt zweimal mit je 400 ml Methanol/Wasser (1:4) gewaschen und anschließend im Vakuum bei 55°C getrocknet. Es wurden 338 g 4-(4-Fluorphenyl)-2,6-diisopropyl-3,5-carbonsäuremethylester-pyridin erhalten, was einer Ausbeute von 90,5 % der Theorie entspricht.

### Beispiel 4

In einem 4 l Glasreaktor wurden 750 g 4-(4-Fluorphenyl)-2,6-diisopropyl-3,5-carbonsäuremethylester-1,4-dihydropyridin in 2250 g Wasser und 245 g Essigsäure vorgelegt. Anschließend wurden bei 58 - 60°C 7 Mol Methylnitrit eingeleitet. Nach Abkühlen auf 20°C wurde mit 363 g 45 gew.-%iger Natronlauge einen pH-Wert von 9 eingestellt. Die dann vorliegende Suspension wurde filtriert, das abfiltrierte Produkt zweimal mit je 800 ml Wasser gewaschen und anschließend im Vakuum bei 50 - 60°C getrocknet. Es wurden 708 g 4-(4-Fluorphenyl)-2,6-diisopropyl-3,5-carbonsäuremethylester-pyridin isoliert, was einer Ausbeute von 95 % der Theorie entspricht.

### Beispiel 5

In einem 4 l Glasreaktor wurden 750 g 4-(4-Fluorphenyl)-2,6-diisopropyl-3,5-carbonsäuremethylester-1,4-dihydropyridin in 1470 g Wasser und 1470 g Essigsäure vorgelegt. Anschließend wurden bei 58 - 60°C 7 Mol Methylnitrit eingeleitet. Nach Abkühlen auf 20°C wurde abgenutscht, das Produkt zweimal mit je 800 ml Wasser gewaschen und anschließend im Vakuum bei 50 - 60°C getrocknet. Es wurden 643 g 4-(4-Fluorphenyl)-2,6-diisopropyl-3,5-carbonsäuremethylester-pyridin isoliert, was einer Ausbeute von 86 % der Theorie entspricht.

## Patentansprüche

1. Verfahren zur Herstellung von substituierten Pyridinen der Formel (I) 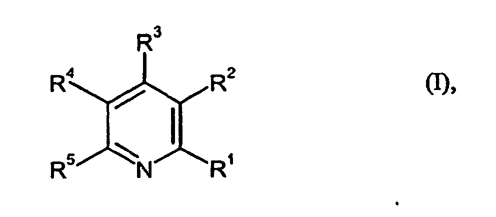 in der
R¹ und R⁵ gleich oder verschieden sind und jeweils für C₁-C₁₀-Alkyl oder C₆-C₁₀-Aryl stehen,
R² und R⁴ gleich oder verschieden sind und jeweils für Wasserstoff, C₁-C₁₀-Alkyl, CN oder COOR⁶ mit R⁶ = C₁-C₁₀-Alkyl stehen und
R³ für Wasserstoff, C₁-C₁₀-Alkyl oder gegebenenfalls durch Halogen, Nitro, COOR⁶ (R⁶ wie oben definiert), CN oder C₁-C₁₀-Alkyl substituiertes C₆-C₁₀-Aryl steht,
**dadurch gekennzeichnet, dass** man ein substituiertes 1,4-Dihydropyridin der Formel (II) 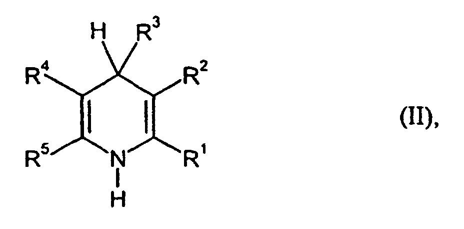 in der
R¹ bis R⁵ die bei Formel (I) angegebene Bedeutung haben,
mit Methylnitrit in Gegenwart von Säure umsetzt, die weniger als 20 Gew.-% oxidierende Bestandteile enthält.

2. Verfahren nach Anspruch 1, **dadurch gekennzeichnet, dass** in den Formeln (I) und (II) R¹ und R⁵ gleich sind und für geradkettiges oder verzweigtes C₁-C₆-Alkyl stehen, R² und R⁴ gleich sind und für COOR⁶ mit R⁶ = geradkettigem oder verzweigtem C₁-C₆-Alkyl stehen und R³ für mit Fluor und/oder Chlor substituiertes Phenyl steht.

3. Verfahren nach Ansprüchen 1 und 2, **dadurch gekennzeichnet, dass** man 4-(4-Fluorphenyl)-2,6-diisopropyl-3,5-carbonsäuremethylester-1,4-dihydropyridin einsetzt und 4-(4-Fluorphenyl)-2,6-diisopropyl-3,5-di(methoxycarbonyl)-methylester-pyridin herstellt.

4. Verfahren nach Ansprüchen 1 bis 3, **dadurch gekennzeichnet, dass** man das benötigte Methylnitrit durch Umsetzung von Alkalinitriten mit Methanol in Gegenwart einer starken Säure herstellt und, bezogen auf 1 Mol 1,4-Dihydropyridin der Formel (II) 1 bis 20 Mol Methylnitrit einsetzt.

5. Verfahren nach Ansprüchen 1 bis 4, **dadurch gekennzeichnet, dass** man als Säure eine Mineral- oder Carbonsäure, bezogen auf 1 Mol 1,4-Dihydropyridin der Formel (II) in einer Menge von 0,01 bis 2 Molen verwendet.

6. Verfahren nach Ansprüchen 1 bis 5, **dadurch gekennzeichnet, dass** man das 1,4-Dihydropyridin der Formel (II) in Form von 1,4-Dihydropyridinium-Salzen von nicht-oxidierenden Säuren einsetzt und Säure nicht separat zusetzt.

7. Verfahren nach Ansprüchen 1 bis 6, **dadurch gekennzeichnet, dass** man es bei Temperaturen im Bereich -30 bis +100°C durchführt.

8. Verfahren nach Ansprüchen 1 bis 7, **dadurch gekennzeichnet, dass** die Säure frei von oxidierenden Bestandteilen ist.

9. Verfahren nach Ansprüchen 1 bis 8, **dadurch gekennzeichnet, dass** man als Säure gasförmigen Chlorwasserstoff, wässrige Salzsäure, wässrige Schwefelsäure oder eine C₁-C₄-Carbonsäure in Substanz oder wässriger Lösung verwendet.

10. Verfahren nach Ansprüchen 1 bis 4 und 6 bis 9, **dadurch gekennzeichnet, dass** man eine Carbonsäure in Mengen von bis zu 50 Mol pro Mol 1,4-Dihydropyridin der Formel (II) einsetzt.

## Claims

1. Process for preparing substituted pyridines of the formula (I) 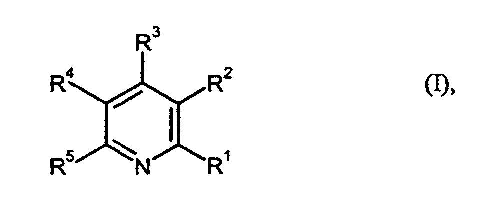 in which
R¹ and R⁵ are identical or different and each represents C₁-C₁₀-alkyl or C₆-C₁₀-aryl,
R² and R⁴ are identical or different and each represents hydrogen, C₁-C₁₀-alkyl, CN or COOR⁶ where R⁶ = C₁-C₁₀-alkyl and
R³ represents hydrogen, C₁-C₁₀-alkyl or represents C₆-C₁₀-aryl which is optionally substituted by halogen, nitro, COOR⁶ (R⁶ is as defined above), CN or C₁-C₁₀-alkyl,
**characterized in that** a substituted 1,4-dihydropyridine of the formula (II) 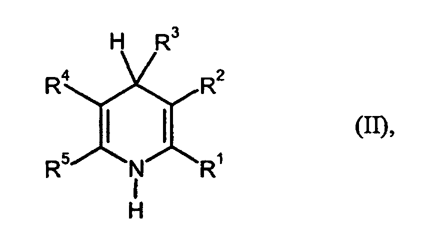 in which
R¹ to R⁵ are as defined under formula (I)
is reacted with methyl nitrite in the presence of acid containing less than 20% by weight of oxidizing components.

2. Process according to Claim 1, **characterized in that** in the formulae (I) and (II) R¹ and R⁵ are identical and represent straight-chain or branched C₁-C₆-alkyl, R² and R⁴ are identical and represent COOR⁶ where R⁶ = straight-chain or branched C₁-C₆-alkyl and R³ represents fluorine- and/or chlorine-substituted phenyl.

3. Process according to Claim 1 or 2, **characterized in that** the starting material used is 4-(4-fluorophenyl)-2,6-diisopropyl-3,5-di(methoxycarbonyl)-1,4-dihydropyridine and the product is 4-(4-fluorophenyl)-2,6-diisopropyl-3,5-di(methoxycarbonyl)-pyridine.

4. Process according to any of Claims 1 to 3, **characterized in that** the methyl nitrite required is prepared by reacting alkali metal nitrites with methanol in the presence of a strong acid, using from 1 to 20 mol of methyl nitrite per mole of 1,4-dihydropyridine of the formula (II).

5. Process according to any of Claims 1 to 4, **characterized in that** the acid used is a mineral or carboxylic acid, in an amount of from 0.01 to 2 mol per mole of 1,4-dihydropyridine of the formula (II).

6. Process according to any of Claims 1 to 5, **characterized in that** the 1,4-dihydropyridine of the formula (II) is employed in the form of 1,4-dihydropyridinium salts of non-oxidizing acids, and the acid is not added separately.

7. Process according to any of Claims 1 to 6, **characterized in that** it is carried out at temperatures in the range from -30 to +100°C.

8. Process according to any of Claims 1 to 7, **characterized in that** the acid is free of oxidizing components.

9. Process according to any of Claims 1 to 8, **characterized in that** the acid used is gaseous hydrogen chloride, aqueous hydrochloric acid, aqueous sulphuric acid or a C₁-C₄-carboxylic acid without diluent or in aqueous solution.

10. Process according to any of Claims 1 to 4 and 6 to 9, **characterized in that** a carboxylic acid is used in amounts of up to 50 mol per mole of 1,4-dihydropyridine of the formula (II).

## Revendications

1. Procédé pour la préparation des pyridines substituées de formule (I) 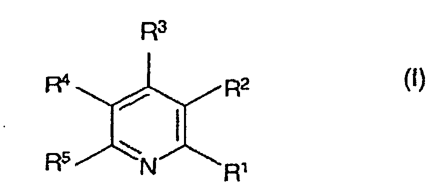 dans laquelle
R¹ et R⁵, ayant des significations identiques ou différentes, représentent chacun un groupe alkyle en C₁ à C₁₀ ou aryle en C₆ à C₁₀,
R² et R⁴, ayant des significations identiques ou différentes, représentent chacun l'hydrogène, un groupe alkyle en C₁ à C₁₀, un groupe CN ou un groupe COOR⁶ pour lequel R⁶ représente un groupe alkyle en C₁ à C₁₀, et
R³ représente l'hydrogène, un groupe alkyle en C₁ à C₁₀ ou un groupe aryle en C₆ à C₁₀ portant éventuellement des substituants halogéno, nitro, COOR⁶ (R⁶ ayant les significations indiquées ci-dessus), CN ou alkyle en C₁ à C₁₀,
**caractérisé en ce que** l'on fait réagir une 1,4-dihydropyridine substituée de formule (II) 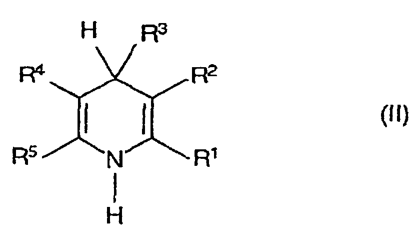 dans laquelle
R¹ à R⁵ ont les significations indiquées en référence à la formule (I),
avec le nitrite de méthyle en présence d'un acide contenant moins de 20 % en poids de constituants oxydants.

2. Procédé selon la revendication 1, **caractérisé en ce que**, dans les formules (I) et (II), R¹ et R⁵ ont des significations identiques et représentent chacun un groupe alkyle à chaîne droite ou ramifiée en C₁ à C₆, R² et R⁴ ont des significations identiques et représentent chacun un groupe COOR⁶ pour lequel R⁶ représente un groupe alkyle à chaîne droite ou ramifiée en C₁ à C₆, et R³ représente un groupe phényle substitué par le fluor et/ou le chlore.

3. Procédé selon les revendications 1 et 2, **caractérisé en ce que** l'on met en oeuvre la 4-(4-fluorophényl)-2,6-diisopropyl-3,5-carboxylate de méthyle-1,4-dihydropyridine et on prépare l'ester 4-(4-fluorophényl)-2,6-diisopropyl-3,5-di(méthoxycarbonyl)-méthylique-pyridine.

4. Procédé selon les revendications 1 à 3, **caractérisé en ce que** l'on prépare le nitrite de méthyle nécessaire par réaction d'un nitrite alcalin avec le méthanol en présence d'un acide fort et on le met en oeuvre en quantité de 1 à 20 mol de nitrite de méthyle par mole de la 1,4-dihydropyridine de formule (II).

5. Procédé selon les revendications 1 à 4, **caractérisé en ce que** l'acide utilisé est un acide minéral ou un acide carboxylique, mis en oeuvre en quantité de 0,01 à 2 mol par mole de la 1,4-dihydropyridine de formule (II).

6. Procédé selon les revendications 1 à 5, **caractérisé en ce que** la 1,4-dihydropyridine de formule (II) est mise en oeuvre à l'état de sel de 1,4-dihydropyridinium d'un acide non oxydant, sans addition séparé d'un acide.

7. Procédé selon les revendications 1 à 6, **caractérisé en ce que** l'on opère des températures dans l'intervalle de -30 à +100°C.

8. Procédé selon les revendications 1 à 7, **caractérisé en ce que** l'acide est exempt de constituants oxydants.

9. Procédé selon les revendications 1 à 8, **caractérisé en ce que** l'acide utilisé consiste en chlorure d'hydrogène gazeux, acide chlorhydrique aqueux, acide sulfurique aqueux ou acide carboxylique en C₁ à C₄ tel quel ou à l'état de solution aqueuse.

10. Procédé selon les revendications 1 à 4 et 6 à 9, **caractérisé en ce que** l'on utilise un acide carboxylique en quantité allant jusqu'à 50 mol par mole de la 1,4-dihydropyridine de formule (II).
